# EUROPEAN PATENT SPECIFICATION

(11) **EP 0 404 968 B1**
(45) Date of publication and mention of the grant of the patent: **30.11.1994**
(21) Application number: 90901900.2
(22) Date of filing: 17.01.1990
(51) Int. Cl.: A61B 17/36

(54) **LASER BEAM EMITTING PROBE AND METHOD OF MANUFACTURING SAME**
LASERSTRAHLSONDE UND VERFAHREN ZUR HERSTELLUNG
SONDE EMETTANT UN FAISCEAU LASER ET PROCEDE DE FABRICATION

(30) Priority: 17.01.1989 JP 18273/89
(43) Date of publication of application: 02.01.1991
(73) Proprietor: S.L.T. JAPAN CO, LTD., Tokyo 102 (JP)
(72) Inventor: DAIKUZONO, Norio, Chiba-ken 290-02 (JP)
(74) Representative: Bloch, Gérard
(86) International application number: PCT/JP90/00040
(87) International publication number: WO 90/07910

(56) References cited:
- EP-A- 0 408 757
- WO-A-85/05262
- JP-A-63 200 750
- JP-A-63 318 934
- JP-U-62 160 918
- US-A- 4 273 127
- PATENT ABSTRACTS OF JAPAN, vol. 12, no. 280 (P-739)[3127], 2nd August 1988;& JP-A-63 60 412 (NIPPON TELEGR. &TELEPH. CORP.) 16-03-1988

## Description

This invention relates to a laser light emitting probe to permit an incision, vaporization of living tissues of an animal such as a human body, a thermal therapy and the like, and the method for producing the laser light emitting probe.

In these days, medical treatments such as incisions of living tissues of animal organisms by irradiation with laser light are conspicuous due to its ability of hemostasis.

It had been the conventional method that the laser light was irradiated from the fore end of an optical fiber which is brought out of contact with the living tissues. But this method causes severe damage to the fore end of the optical fiber. Therefore, a method which has been utilized lately is as follows;

First, laser light is transmitted into an optical fiber, whose fore end portion locates adjacent to treated living tissues. Next, the laser light fed out from the optical fiber is fed into an emitting probe, which is brought into or out of contact with the living tissues. Then, the laser light is emitted from the surface of the probe to be irradiated against the living tissues. In this case, the probe should be brought into contact with the living tissues (hereafter "living tissue" is sometimes expressed by "tissue" only).

On a medical treatment, if a probe is brought into contact with living tissues, there is an advantage that laser light can be irradiated against a precise position with small power loss comparing with a case, in which the probe is brought out of contact with the living tissues.

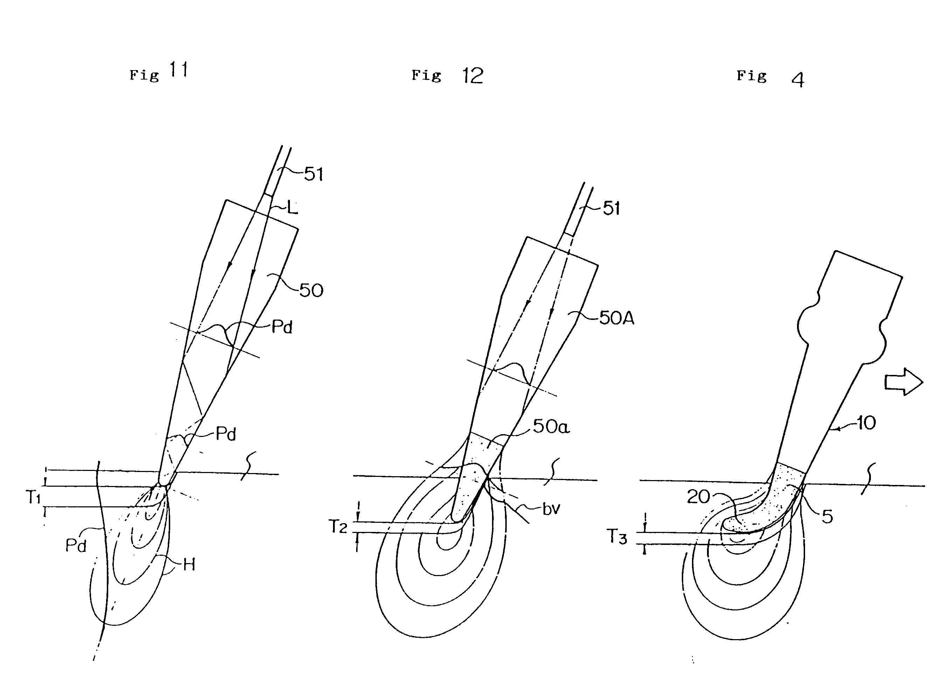

Therefore, the inventor developed many kinds of contact probes which are utilized for various purposes. One embodiment is shown in Fig. 11 which probe is disclosed in WO-A-85/05262. This probe is made of sapphire, quartz and the like. Usually, it has a smooth surface and is not provided with a surface layer. Further, it has a fore end portion of a uniformly tapered conical shape.

Referring to Fig. 11, laser light L goes through an optical fiber 51 and is fed into the probe 50, which is of long and narrow conical shape with a round fore end portion and whose outer surface is smooth. The laser light L passing through the probe 50 is reflected and refracted on an inner surface of the probe 50 to reach its tip end. Finally the laser light L is emitted only from the tip end concentratedly.

In this case, a power density and its distribution diagram of laser light irradiation are shown as contour lines H and a curve Pd in Fig. 11 respectively. Accordingly, it is obvious that the laser light L is concentratedly emitted from the tip end of the probe 50. Therefore, while the thickness (depth) T1 of a coagulation layer C is increased, the laser light L is not irradiated effectively against tissues along the side of the probe, then the effect of an incision and hemostasis there is reduced.

As a result, in case of the incision of hemorrhagic tissues such as liver and the like, the probe must be repeated to move along the same incision line little by little. Therefore, the surgery requires much labour and must be done carefully.

Under these circumstances, the inventor found that by forming a rough surface on a probe, the effective area of laser light irradiation can be extended, because laser light is refracted on the rough surface to be emitted in many directions.

Although it is possible to extend the effective area of the laser light irradiation by means of the above mentioned method, its effect is not sufficient. In order to compensate the low effect of an incision for tissues along the side of the probe, an output power level from a laser light generator should be enlarged. Further, the laser light having a high power level causes a severe damage to the tissues. Therefore, this probe is not applicable for the incision of hemorrhagic internal organs.

The inventor had done a lot of researches on these defects to find the following things.

The effect of an incision at a side part of an incised portion is enlarged by forming a rough surface on a probe and a surface layer having laser light scattering particles on the rough surface of the probe. Such a probe is disclosed e.g. in EP-A-0 292 621. The scattering effect is enlarged by means of the light scattering particles in the surface layer as well as the rough surface of the probe.

Referring to Fig.12, by the probe 50A having the surface layer 50a, a broad area of the laser light irradiation can be obtained, while a depth T2 of a coagulation layer is reduced. Accordingly, the effect of the incision at the side part of the incised tissue is enlarged.

However, there are still some defects with the probe. The first defect relates to hemostasis. Since the power level of the laser light irradiation from the tip end is higher than that from the side of the probe, the coagulation layer in the tissues at the side part of the probe is not so deep. Therefore, the effect of the hemostasis directed by the depth of the coagulation is known to be reduced at the side part. If there is a blood vessel by adjacent to the thin coagulation at the side part of the probe, there is a fear of bleeding there.

The second defect relates to the process of the incision. When the probe starts to move along an incision line, the tissues are incised by the tip end of the probe. Therefore, it is comparatively easy for the probe to move on the surface of the tissues. However, there remains a difficulty of a probe-movement in the tissues at the side part of the probe. In other words, it is easy to start to incise, however, it is difficult to continue to incise along the incision line and since the probe moves too easily on the surface of the tissues, it is also difficult to know an incised depth in the tissues.

On the other hand, such consideration for the hemostasis must not be taken for the incision of less hemorrhagic tissues such as skin, fat layer and the like. However, in order to compensate the reduced effect of the incision at the side part of the incised tissues, the laser light having a high power level is required. This causes a damage to the tissues. Further, this necessitates a high power and expensive laser light generator. If the power level remains a low level, the probe must be moved slowly, thus, it takes a long time to carry out a surgery with this probe.

A laser light emitting probe comprising, longitudinally, a base end on which laser light is impinged, a body through which said laser light is penetrated and a fore end portion from whose external surface said laser light is emitted, said fore end portion being provided with a surface layer on the surface of at least from the bending part to the tip end of said bending fore end portion, is already known from JP-U-62160918. This reference relates to a flexible probe the tip of which bends at a certain angle when under pressure. The surface of the tip of this probe is sandblasted to scatter the laser light.

A laser light emitting probe comprising in its surface layer laser light absorbing particles and laser light scattering particles having a refractive index larger than that of the probe material is disclosed in the intermediate European application EP-A-0 408 757. This laser probe, however, is not bent.

US-A-4 273 127 relates to a laser probe for cutting and coagulating tissue. The probe includes a transparent light guide having a narrow working surface or edge. As the light propagates toward the tapered working edge its divergence is increased such that when it exits the edge of the probe, the radiation is widely spread which for tissue coagulation. The edge may also be curved to further enhance dispersion of the radiation.

Therefore, it is a main object of the present invention to provide a laser light emitting probe permitting following things;
laser light irradiation with a broad effective area;
an effective incision by vaporization;
an effective hemostasis at a side part of incised tissues;
an incision for less-hemorrhagic tissues with a low power level of laser light irradiation; and
an incision by quick and easy probe-movement for hemorrhagic tissues and also for the less-hemorrhagic tissues.

To this end, the invention relates to a probe of the above mentioned type, characterized by the fact that said fore end portion bends at a certain angle on a longitudinal cross section, said angle, made by crossing a center line of a straight portion and a normal line, which is perpendicular to a tangent line touching an externally swelled surface of the bending part of said fore end portion, being larger than a critical angle of the material of said probe to the air and said surface layer containing laser light absorbing particles and laser light scattering particles having a larger refractive index than that of the material of said probe.

The present invention also relates to a method for producing the laser light emitting probe, comprising the steps of;
tapering a laser light transmissible member at least at its fore end portion and towards an external side on a cross section,
bending the fore end portion of the tapered transmissible member at a certain angle on a longitudinal cross section;
bringing the bending transmissible member into contact with a dispersion containing at least laser light absorbing particles, laser light scattering particles having a larger refractive index than that of the material of the transmissible member and laser light transmissible particles which have a melting point same as or lower than that of the transmissible member, and
baking the contacted transmissible member with the dispersion at a temperature which is higher than the melting point of the laser light transmissible particles and within a limit so that the transmissible member can keep its shape.

According to the present invention, by forming a laser light scattering surface on the fore end portion of the probe, the laser light can be irradiated with a broad effective area. Because, when the laser light is penetrated through the probe, the laser light is scattered at the surface of the probe. Therefore, the power level of the laser light emitted from the tip end of the probe is reduced while the power level of the laser light from the side surface of the probe is increased. Further, since the probe has the fore end portion, which bends at a certain angle on a longitudinal section, the power density distribution of the laser light is concentrated at the externally swelled side part of the bending fore end portion than at the inner side part thereof where there is a curvature center of bending. Simultaneously, the laser light having a high power level is irradiated from the side surface of the probe against the tissues.

Therefore, vaporization of the tissues, which are to be incised by the side of the probe, is accelerated, thus, the tissues can be incised easily. Further, as the depth of a coagulation layer caused by the incision is increased, even hemorrhagic tissue can be incised easily without much bleeding.

Instead of forming a light scattering surface on a laser light transmissible member only by roughening, a surface layer is formed on the surface of the transmissible member, which contains light scattering particles, preferably made of sapphire and the like and which have a larger refractive index than that of the transmissible member . When laser light emitted from the transmissible member passes through the surface layer , the laser light impinges on the light scattering particles to be partially reflected on the surface of the light scattering particles , or to be partially penetrated into and emitted from the particles with refraction. Therefore, the laser light is emitted in various directions from the entire surface layer . As a result, by forming the surface layer , the area of laser light irradiation can be extended to be larger than the area of laser light irradiation in case of forming the light scattering surface only by roughening.

Further, the surface layer contains laser light absorbing particles, preferably made of carbon and the like. When the laser light is impinged on the laser light absorbing particles , the greater part of the energy of the laser light is converted to heat energy by means of the laser light absorbing particles , and the tissues are heated by the heat energy from the surface layer .

Therefore, as the vaporization of the tissues is accelerated, the tissues can be incised with the laser light, which has low power level and which is penetrated into the transmissible member . Accordingly, when the tissues are incised, the transmissible member can be moved rapidly. Further, the required energy of the laser light penetrating into the transmissible member is low. As a result, the surgery can be carried out in short time, further with a cheap and small scaled laser light generator.

Preferably, the surface of the transmissible member is roughened, then the above mentioned surface layer is formed on the rough surface to give a large effect of laser light scattering.

On the other hand, referring to the surface layer, for example, if a dispersion containing the above mentioned laser light absorbing particles and the light scattering particles is coated on the surface of the transmissible member, after the vaporization of a dispersion medium, the contact of the probe having the surface layer with the tissue or other substances causes a damage to the surface layer. Because the both kinds of particles are attached to the surface of the transmissible member only by physical adsorptive power.

Therefore, by a binder which sticks the laser light absorbing particles and the light scattering particles to the surface of the transmissible member, adhesion of the surface layer to the transmissible member is enlarged.

In this case, the binder is preferably made of light transmissible material such as quartz and the like to ensure the emission of the laser light from the surface layer . The laser light transmissible particles, which have a melting point same as or lower than that of the transmissible member , are used as the transmissible material and they are dispersed together with the absorbing particles and the light scattering particles in a proper liquid such as water. Then, the transmissible member painted with this dispersion is baked at a temperature which is higher than a melting point of the transmissible particle and within a limit as the transmissible member can keep its shape. As a result, the transmissible particles melt to form the surface layer of high mechanical strength together with the laser light absorbing particles and the light scattering particles. Therefore, the damages to the surface layer can be reduced because of its high strength.

The present invention shall be better understood with reference to the accompanying drawings.

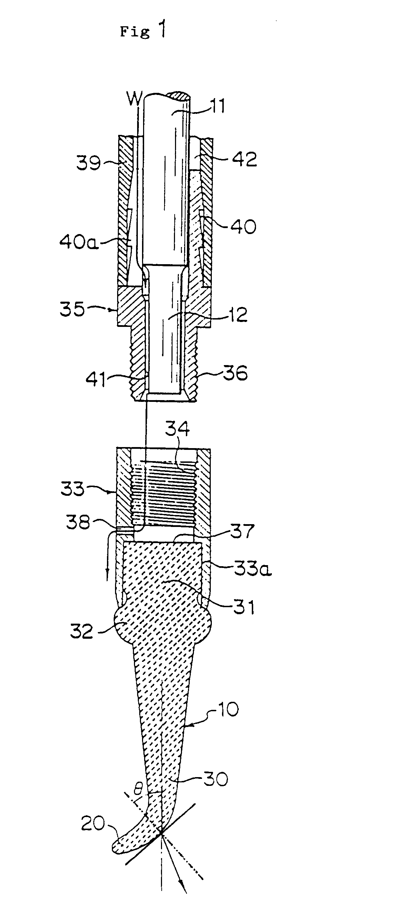

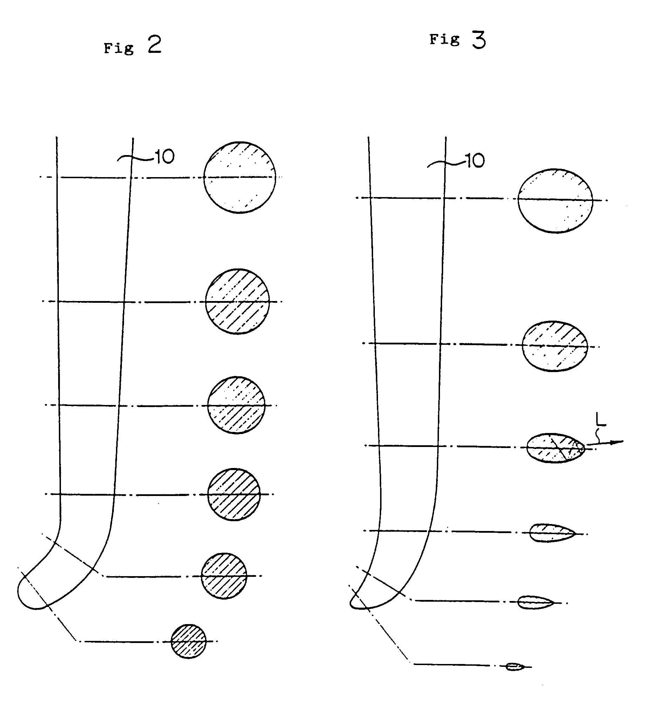

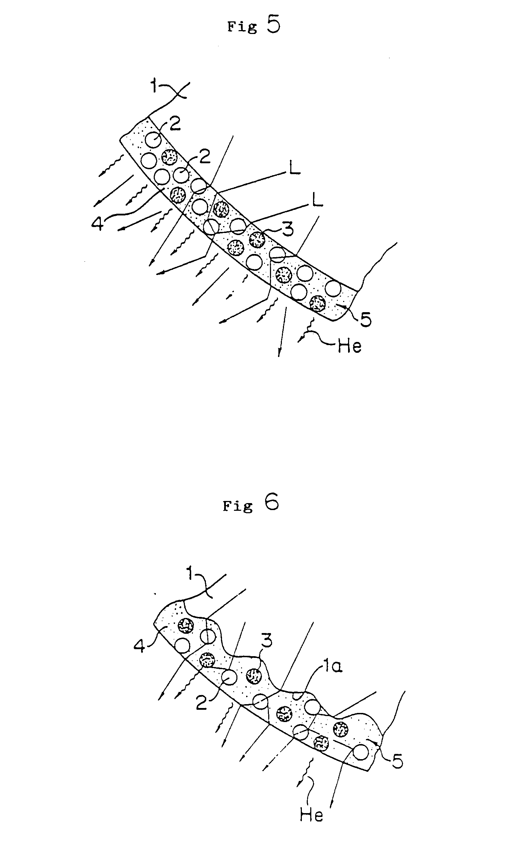

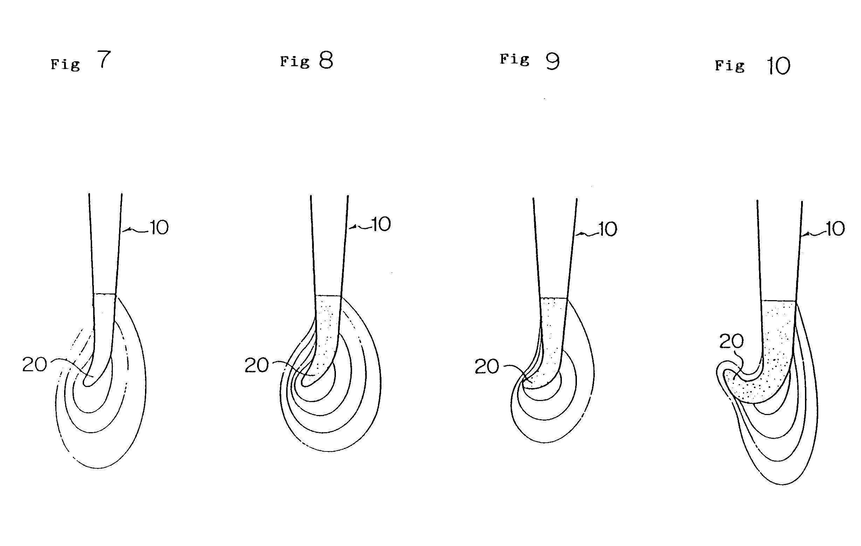

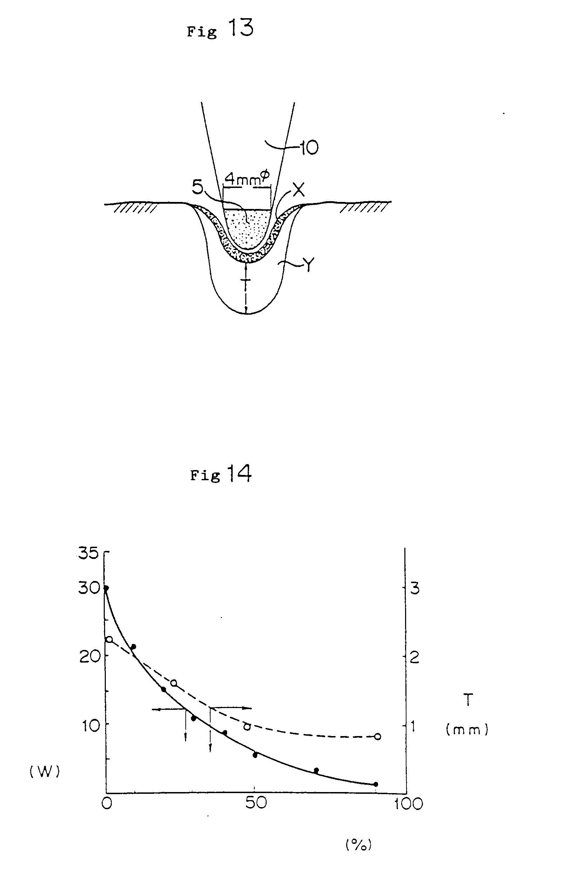

Fig. 1 is a longitudinal sectional view of a structure of a probe and a holding member therefor relating to the present invention; Figs. 2 and 3 are schematic enlarged front views and enlarged sectional views of the important parts of laser light emitting probes relating to the present invention; Fig. 4 is a schematic illustration showing an embodiment of a laser light emitting probe for a laser scalpel and a power density distribution diagram of laser light irradiation in this embodiment; Figs. 5 and 6 are enlarged sectional views of surface layers: Figs. 7, 8, 9 and 10 are power density distribution diagrams of laser light irradiation, where the fore end portion of each probe is bending at an each angle; Fig. 11 is a schematic illustration showing a power density distribution diagram of laser light irradiation with a conventional probe; Fig. 12 is a schematic illustration showing a power density distribution diagram of laser light irradiation with another conventional probe for comparing; Fig. 13 is a schematic illustration explaining an experiment; Fig. 14 is a graph showing the result of the experiment of Fig. 13.

Fig. 5 is an enlarged sectional view of a surface layer 5 formed on a probe 10, whose structure is shown in Fig. 1 as an embodiment. On the transmissible member 1, the surface layer 5 containing laser light scattering particles 2 and laser light absorbing particles 3 is formed with a laser light transmissible material 4 as a binder made from melted laser light transmissible particles as described before.

In this case, by forming a rough surface 1a on the transmissible member 1, as shown in Fig. 6, a scattering effect of laser light irradiation is enlarged.

An example of the structure of the probe 10 and a holding member therefor is shown in Fig. 1. This probe 10 comprises a conically tapered inserting portion 30, a main holding portion 31, a flange 32 formed between them, and a bending fore end portion 20. The main holding portion 31 of the probe 10 is fitted in a cylindrical female connector 33 and fixed integrally thereto by caulking the mating portions 33a, by using a ceramic type adhesive between the mating surfaces or by the combination of these both means. The female connector 33 has, on the internal side surface thereof a female screw 34, which is adapted to mate removably with a male screw 36 of a male connector 35. The female connector 33 has holes 38 which facilitates the passage of cooling water W inside and outside thereof. The holes 38 are disposed adjacent to the top of a light receiving base 37 of the probe 10, for example, oppositely on a circumference of the female connector 33 although only one of them is shown in Fig. 1. Then, the male connector 35 is pressed to be fitted into the end portion of a flexible tube jacket 39 fabricated of, for example, Teflon (the trademark for polytetrafluoroethylene). For this press fitting, the male connector 35 has stepped portions 40 at its base portion, and is firmly held so as not to be disengaged from the tube jacket 39.

A laser light transmitting optical fiber 11 attached by an optical fiber 12 is inserted in the tube jacket 39 and the male connector 35. There is a gap 42 between the optical fiber 11 and the tube jacket 39 for passage of the cooling water. Although the end portion of the transmitting optical fiber 11 is closely fitted in the male connector 35 at its stepped portion 40, the stepped portion 40 has, for example, two slits 40a formed oppositely on a circumference of the stepped portion 40 for the cooling water W. The cooling water W is passed through the slits 40a and a passage 41 provided between the inner surface of the fore end portion of the male connector 35 and the outer surface of the transmitting optical fiber 11.

A laser light emitting apparatus of this type described above is equipped in an endoscope and some other suitable holders while the female connector 33 is connected to mate with the male connector 35. Alternatively, the female connector 33 attached by the probe 10 is equipped directly in some suitable holders which already built in the transmitting optical fiber 11.

In this way, a pulse laser light such as YAG and the like introduced through the optical fiber 12 is fed into the probe 10 from the light receiving base 37, therefore, is emitted mainly from all over the outer surface of the bending fore end portion 20. At the same time, the cooling water W is fed through the gap 42, the slit 40a, the gap 41 to cool the probe 10, further, discharged through the opening 38 to flow out on the surface of tissues and then cool the tissues.

The transmissible member of this invention is preferably fabricated from a natural or artificial ceramic material such as diamond, sapphire, quartz and the like due to their heat resistance.

The light scattering particles relating to the present invention, having a larger refractive index for the laser light than that of the transmissible member are of a natural or artificial material such as diamond, sapphire, quartz (a melting point is preferably high), single crystal zirconium oxide (ZrO₂), high melting point glass, transmissible and heat resistant synthetic resin, laser light reflective metal such as gold or aluminium, and a particle which is laser light reflective or non-reflective metal particle coated with laser reflective metal such as gold, aluminum and the like by means of the surface treatment such as plating.

The laser transmissible material is preferably made of transmissible particle which can make a film when it melts, and more preferably has heat resistance, for example, a natural or artificial, sapphire, quartz, glass, transmissible and heat resistant synthetic resin and the like. A suitable transmissible material is selected from these materials in consideration of the relation to the material of the transmissible member.

The laser light absorbing particle is made of carbon, graphite, iron oxide, manganese dioxide and the any other materials which can absorb the laser light to generate heat energy.

A content of each particle in the surface layer(wt%) and each average particle size is preferably within ranges as shown in a following table. More preferable content and particle size are put in parentheses.

| | Content(wt%) | Average Particle Size(µm) |
|---|---|---|
| Light Scattering Particle(A) | 90 - 1 (70 - 20) | 0.2-300 (1 - 50) |
| Transmissible Particle(B) | 10 - 90 (20 - 50) | 0.2 - 500 |
| Absorbing Particle(C) | 90 - 1 (70 - 10) | 0.2 - 500 (1 - 100) |

The thickness of the surface layer is preferably 10µm - 5mm, more preferably 30µm - 1mm. If the surface layer having a desired thickness can not be formed by one cycle of the method, this cycle should be repeated until the surface having the desired thickness can be obtained. The surface layer is formed by following methods;

The above mentioned three kinds of particles are dispersed in a dispersion medium, then it is heated to a temperature which is higher than the melting point of the transmissible particles, and the transmissible member is dipped in the dispersion.

Alternatively the above mentioned three kinds of particles are melted to be sprayed to the transmissible member simultaneously.

Further, other suitable methods for forming the surface layer can be used.

By the above described first method, the dispersion dispersing the three kinds of particles can be painted to the transmissible member. Moreover, this painting method facilitates the operation, because what should be done in this method is that only a part, which is desired to be covered with the surface layer, of the transmissible member, or preferably only a part, which is desired to be the bending fore end portion, is dipped in the dispersion and pulled up therefrom. Therefore this method is practical and rational.

As the dispersion medium, suitable liquid for example, water, alcohol or mixture of them can be used. Further sugar or starch is added to increase the viscosity of the dispersion medium.

As described before, according to the present invention, by forming the surface layer 5 on the surface of the transmissible member 1, the area of the laser light irradiation on the tissues is extended, because, the laser light is emitted widely in many directions from the surface layer 5 as shown in Fig. 4 and as described above.

On the other hand, the inventor performed an experiment as follows, using a probe, whose fore end portion is not bending but straight as shown in Fig. 13;

In a following section, the contents of the light scattering particle, that of the transmissible particle, and that of the laser light absorbing particle will be referred as (A), (B) and (C) respectively. The inventor investigated each change of following two parameters against (C) under fixed condition of (A) : (B) = 2 : 1. One investigated parameter is a laser light power level, with which an incision to a pig's liver can be started. Another parameter is a depth T of a coagulation layer Y below a carbonized layer X of treated tissues. Then, from the result in Fig. 14, following things are known.

When the percentage of (C) is high, the incision can be started with the laser light having a low power level, then, it is possible for the probe to be moved quickly. The effect of hemostasis can be known by the depth d of the coagulation. Therefore, since the depth d is decreased, the hemostasis of the treated tissues is turned out to be reduced. Finally, the probe with high percentage of (C) in the surface layer can be used effectively for the incision to the tissues which bear the damage to some extent such as skin, fat layer and the like.

On the other hand, the probe with low percentage of (C) can be used efficiently for an incision of the tissues, for which the hemostasis is regarded to be important. Such tissues are, for example, liver, heart and the like. In this case, it is clear that the power level of the output from the laser light generator must be raised and the probe must be moved slowly.

Referring to this experiment and the like, the inventor introduced these two equations, (1) and (2). 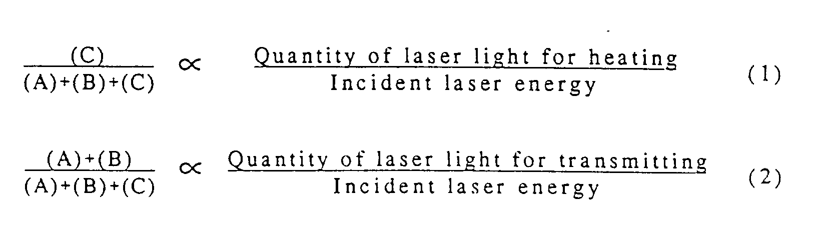
Equation (1) means that heat generation is progressed as (C) is increased. Accordingly, the incision is carried out by mainly vaporization. Therefore, since most of the incident laser energy is spent for the heating, the laser light can not penetrate so deeply into the tissues. As a result, the depth of the coagulation layer is reduced.

Equation (2) means that the penetration of the laser light into the tissues is progressed as (C) is decreased. Therefore, the tissue absorbing laser light is heated, thus, the coagulation is made in the tissues.

Accordingly, if some kinds of probes, which differs in only percentage of (C) in the surface layers, are prepared in advance, a suitable probe can be selected in accordance with a medical purpose, thereby a suitable treatment can be carried out easily.

In the present invention, although the probe must be covered with the light scattered surface, the surface layer is not necessary. That is to say, the probe may have only the rough surface as the scattering surface.

However, the probe in the present invention must have the fore end portion, which bends at a certain angle on a longitudinal section. In this case, the meaning of "bending" includes the operation of making an angle straightly as well as the operation of making a curve.

The power density distribution of the laser light irradiation varies with the change of a bending angle as shown in Figs. 7, 8, 9 and 10. When the bending angle is increased, the rate of the power level of laser light emission from the side surface of the probe is increased. In the present invention, the bending angle ϑ shown in Fig. 1 is made by crossing the center line of a straight portion of the probe and a normal line. This normal line is perpendicular to the tangent line touching the externally swelled surface of the bending part of the fore end portion on the longitudinal section. The angle ϑ is preferably larger than the critical angle of the material of the probe (the transmissible member) to the air to give a sufficient effect of the laser light scattering from the side surface of the probe.

When the thickness of the bending fore end portion is uniform, the laser light emitted from the tip end is increased while that from the side surface of the probe is decreased. Therefore, the bending fore end portion is preferably tapered.

If the cross section of the probe is circle as shown in Fig. 2, the laser light emitted from the circumference of the probe is substantially uniform. However, as shown in Fig. 3, if the externally swelled side of the bending fore end portion is tapered towards an external side (to the right or lower right on Fig. 3), the laser light emission toward the external side is increased. This is obvious from the illustration of a laser light course in Fig. 3. The incision is, thereby, carried out much easily. Moreover, the probe which is sharp-edged towards the external side facilitates a mechanical incision as well as the incision with the laser light irradiation. As a result, the effect of the incision is significantly enlarged.

Now, an example of producing method for this probe is explained;
First, a conically tapered probe is heated to a temperature higher than a softening temperature of the probe.

Next, only the part of the fore end portion of the probe is bent at a certain angle on a longitudinal section.

Finally, a part of an externally swelled side of the bending fore end portion is pressed from both before and back outer surfaces of the fore end portion so that the above mentioned probe can be formed.

If the surface layer is formed on the surface of the probe having the bending fore end portion, the probe shows the above mentioned characteristics more clearly.

The shape of the base portion of the probe in the present invention is not limited to the cylindrical shape in Figs. 1 and 4. It also comprises shapes such as tapered cone.

It is apparent from the foregoing explanation that the probe of the present invention, which is brought into contact with the living tissues, can be applied suitably for the incision, the vaporization and the coagulation of the living tissues, the thermal therapy and the like.

## Claims

1. A laser light emitting probe (10) comprising, longitudinally, a base end (37) on which laser light is impinged, a body (31) through which said laser light is penetrated and a fore end portion (20) from whose external surface said laser light is emitted, said fore end portion (20) being provided with a surface layer (5), said fore end portion (20) bends at a certain angle (ϑ) on a longitudinal cross section, said angle, made by crossing a center line of a straight portion and a normal line, which is perpendicular to a tangent line touching an externally swelled surface of the bending part of said fore end portion (20), being larger than a critical angle of the material of said probe (10) to the air and said surface layer (5) containing laser light absorbing particles (3) and laser light scattering particles (2) having a larger refractive index than that of the material (1) of said probe on the surface of at least from the bending part to the tip end of said bending fore end portion (20).

2. A laser light emitting probe (10) according to claim 1, wherein said surface layer (5) contains said laser light absorbing particles (3), said laser light scattering particles (2) and a binder (4) made from a laser light transmissible material.

3. A laser light emitting probe (10) according to claim 1 or 2, wherein a rough surface (1a) is formed on the surface of at least said bending fore end portion (20) of said probe (10) and said surface layer (5) is formed on said rough surface (1a).

4. A laser light emitting probe (10) according to one of claims 1 to 3, wherein an externally swelled surface of said bending fore end portion (20) is tapered towards an external side on a cross section.

5. A method for producing a laser light emitting probe (10) according to claim 1, comprising the steps of
(a) tapering a laser light transmissible member (1) at least at a fore end portion (20) thereof and towards an external side on a cross section,
(b) bending said tapered fore end portion (20) of said transmissible member (1) at a certain angle on a longitudinal cross section,
(c) bringing said bending transmissible member (1) into contact with a dispersion containing at least laser light absorbing particles (3), laser light scattering particles (2), which have a larger refractive index than that of said transmissible member (1), and laser light transmissible particles (4) which have a melting point same as or lower than that of said transmissible member (1) and
(d) baking said contacted transmissible member (1) and said dispersion at a temperature which is higher than the melting point of said laser light transmissible particles (4) and within a limit so that said transmissible member (1) can keep its shape.

## Patentansprüche

1. Laserstrahlsonde (10), welche in Längsrichtung ein Basis-Ende (37), auf das Laserlicht auftrifft, einen Körper (31), den das Laserlicht durchdringt, und einen vorderen Endteil (20) umfaßt, von dessen äußerer Oberfläche dieses Laserlicht ausgesendet wird, wobei der vordere Endteil (20) mit einer Oberflächenschicht (5) versehen ist, sich der vordere Endteil (20) im Längsschnitt mit einem bestimmten Winkel (ϑ) biegt, wobei dieser Winkel, erhalten durch das Kreuzen einer Mittellinie eines geraden Teils und einer Normalen, die senkrecht zu einer Tangente ist, welche eine sich ausbauchende Oberfläche des sich biegenden Teils dieses vorderen Endteils (20) berührt, größer ist als ein Grenzwinkel des Materials der Sonde (10) zur Luft und die Oberflächenschicht (5) auf der Oberfläche mindestens von dem sich biegenden Teil bis zum Spitzenende des sich biegenden vorderen Endteils (20) Laserlicht absorbierende Partikel (3) und Laserlicht streuende Partikel (2) mit einem größeren Brechungsindex als das Material (1) der Sonde enthält.

2. Laserstrahlsonde (10) nach Anspruch 1, bei der die Oberflächenschicht (5) die das Laserlicht absorbierenden Partikel (3), die das Laserlicht streuenden Partikel (2) und ein Bindemittel (4) aus einem laserlichtdurchlässigen Material enthält.

3. Laserstrahlsonde (10) nach Anspruch 1 oder 2, bei der auf der Oberfläche von mindestens dem sich biegenden vorderen Endteil (20) der Sonde (10) eine rauhe Oberfläche (1a) ausgebildet ist und die Oberflächenschicht (5) auf dieser rauhen Oberfläche (1a) gebildet ist.

4. Laserstrahlsonde (10) nach einem der Ansprüche 1 bis 3, bei der eine sich ausbauchende Oberfläche des sich biegenden vorderen Endteils (20) im Querschnitt gesehen zu einer Außenseite hin zugespitzt ist.

5. Verfahren zur Herstellung einer Laserstrahlsonde (10) nach Anspruch 1, umfassend die folgenden Schritte:
(a) das Zuspitzen eines laserlichtdurchlässigen Elementes (1) zumindest an einem vorderen Endteil (20) desselben und im Querschnitt gesehen zu einer Außenseite hin,
(b) das Biegen des zugespitzten vorderen Endteils (20) des durchlässigen Elementes (1) mit einem bestimmten Winkel in bezug auf einen Längsschnitt,
(c) das Inkontaktbringen des gebogenen durchlässigen Elementes (1) mit einer Dispersion, die zumindest Laserlicht absorbierende Partikel (3), Laserlicht streuende Partikel (2), welche einen größeren Brechungsindex aufweisen als das durchlässige Element (1), und laserlichtdurchlässige Partikel (4) enthält, die einen Schmelzpunkt besitzen, der dem des durchlässigen Elementes (1) entspricht oder niedriger als dieser ist, und
(d) das Brennen dieses durchlässigen Elementes (1) und der Dispersion, welche sich in Kontakt befinden, bei einer Temperatur, die höher ist als der Schmelzpunkt der laserlichtdurchlässigen Partikel (4) und die innerhalb einer Grenze liegt, so daß das durchlässige Element (1) seine Form beibehalten kann.

## Revendications

1. Sonde émettrice de lumière laser (10) comprenant, longitudinalement, une extrémité de base (37) frappée par la lumière laser, un corps (31) traversé par la lumière laser et une partie extrême avant (20) de la surface extérieure de laquelle la lumière laser est émise, cette partie extrême avant (20) étant pourvue d'une couche superficielle (5), cette partie extrême avant (20) étant courbée à un certain angle (ϑ) sur une section longitudinale, cet angle, formé par l'axe d'une partie rectiligne et par une normale perpendiculaire à une tangente à une surface renflée extérieurement de la partie courbée de la partie extrême avant (20), étant supérieur à l'angle limite de la matière de la sonde (10) à l'air, et la couche superficielle (5) contenant des particules absorbant la lumière laser (3) et des particules diffusant la lumière laser (2) ayant un indice de réfraction supérieur à celui de la matière (1) de la sonde au moins sur sa surface allant de sa partie courbée à son extrémité.

2. Sonde émettrice de lumière laser (10) selon la revendication 1, dans laquelle la couche superficielle (5) contient lesdites particules absorbant la lumière laser (3), les particules diffusant la lumière laser (2) et un liant (4) constitué d'une matière transmettant la lumière laser.

3. Sonde émettrice de lumière laser (10) selon l'une des revendications 1 et 2, dans laquelle une surface rugueuse (1a) est formée sur la surface d'au moins la partie extrême avant courbée (20) de ladite sonde (10) et ladite couche superficielle (5) est formée sur ladite surface rugueuse (1a).

4. Sonde émettrice de lumière laser (10) selon l'une des revendications 1 à 3, dans laquelle une surface renflée extérieurement de la partie extrême avant courbée (20) est effilée vers un côté extérieur sur une section.

5. Procédé de production d'une sonde émettrice de lumière laser (10) selon la revendication 1, comprenant les étapes suivantes :
(a) effilage d'un élément transmettant la lumière laser (1) au moins à une partie extrême avant (20) de celui-ci et vers un côté extérieur sur une section,
(b) courbage de la partie extrême avant effilée (20) de l'élément transmetteur (1) à un certain angle sur une section longitudinale,
(c) mise de l'élément transmetteur courbé (1) en contact avec une dispersion contenant au moins des particules absorbant la lumière laser (3), des particules diffusant la lumière laser (2) ayant un indice de réfraction supérieur à celui de l'élément transmetteur (1) et des particules transmettant la lumière laser (4) ayant un point de fusion égal ou inférieur à celui de l'élément transmetteur (1) et
(d) cuisson de l'élément transmetteur (1) mis en contact et de la dispersion à une température supérieure au point de fusion des particules transmettant la lumière laser (4) et dans des limites telles que ledit élément transmetteur (1) puisse conserver sa forme.
